(19) Europäisches Patentamt
European Patent Office
Office européen des brevets

(11) EP 3 667 318 A1

(12) EUROPEAN PATENT APPLICATION
published in accordance with Art. 153(4) EPC

(43) Date of publication:
17.06.2020 Bulletin 2020/25

(51) Int Cl.:
*G01N 33/53* (2006.01)     *A61K 39/395* (2006.01)
*A61P 35/00* (2006.01)

(21) Application number: 18844044.0

(22) Date of filing: 06.08.2018

(86) International application number:
PCT/JP2018/029390

(87) International publication number:
WO 2019/031444 (14.02.2019 Gazette 2019/07)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 07.08.2017 JP 2017152735

(71) Applicant: Kyushu University, National University
Corporation
Fukuoka-shi, Fukuoka 819-0395 (JP)

(72) Inventors:
• MATSUMOTO Koichiro
Fukuoka-shi
Fukuoka 8190395 (JP)
• YANAGIHARA Toyoshi
Fukuoka-shi
Fukuoka 8190395 (JP)
• TANAKA Kentaro
Fukuoka-shi
Fukuoka 8190395 (JP)

(74) Representative: EP&C
P.O. Box 3241
2280 GE Rijswijk (NL)

(54) METHOD FOR MEASURING OCCUPANCY RATE OF SPECIFIC BINDING SUBSTANCES IN CELL POPULATION

(57) A method for measuring an occupancy rate of a first specific binding substance in a cell population, including bringing a second and a third specific binding substance into contact with the cell population and counting the number of cells to which the second and the third specific binding substances binds, in which all of the first, the second, and the third specific binding substances are specific binding substances that bind to a target cell surface protein, the occupancy rate is a proportion of cells to which the first specific binding substance binds, in cells expressing the target cell surface protein, the second specific binding substance competes with the first specific binding substance, the third specific binding substance does not compete with the first specific binding substance, and the occupancy rate (%) is a value calculated by an expression: (1 - (Number of cells to which the second specific binding substance binds/Number of cells to which the third specific binding substance binds)) × 100.

EP 3 667 318 A1

**Description**

TECHNICAL FIELD

The present invention relates to a method for measuring an occupancy rate of a specific binding substance in a cell population. More specifically, the present invention relates to a method for measuring an occupancy rate of a specific binding substance in a cell population that is brought into contact with the specific binding substance, a kit for measuring an occupancy rate of a specific binding substance in a cell population that is brought into contact with the specific binding substance, a method for obtaining data for optimizing an administration interval or a dosage of an antibody drug to a patient, and a method for obtaining data for optimizing an administration interval or a dosage of nivolumab or pembrolizumab to a patient.

Priority is claimed on Japanese Patent Application No. 2017-152735, filed on August 7, 2017, the content of which is incorporated herein by reference.

BACKGROUND ART

Nivolumab is an antibody drug that specifically binds to programmed death-1 (PD-1) present on the cell surface. Immune checkpoint inhibitors represented by nivolumab have been approved for malignant melanoma, non-small cell lung cancer, renal cell carcinoma, Hodgkin's lymphoma, and head and neck cancer, and have received public attention due to their high therapeutic effects.

On the other hand, immune checkpoint inhibitors are known to have very high drug costs and to cause serious immunity-related adverse events in some patients. For this reason, there is a demand for a technique for analyzing the dynamics of an immune checkpoint inhibitor. For example, NPL 1 has reported the results of examining the dynamics of nivolumab in the body.

Citation List

Non-Patent Literature

[NPL 1] Brahmer J. R., Phase I study of single-agent anti-programmed death-1 (MDX-1106) in refractory solid tumors: safety, clinical activity, pharmacodynamics, and immunologic correlates., J. Clin. Oncol., 28 (19), 3167-3175, 2010.

DISCLOSURE OF INVENTION

Technical Problem

However, since the method described in NPL 1 is complicated and it is necessary to use an expensive antibody preparation, the general spread of the method is considered to be difficult. An object of the present invention is to provide a technique for simply measuring the dynamics of a specific binding substance.

Solution to Problem

The present invention includes the following aspects.

[1] A method for measuring an occupancy rate of a first specific binding substance in a cell population, including bringing a second specific binding substance into contact with the cell population, bringing a third specific binding substance into contact with the cell population, counting the number of cells to which the second specific binding substance binds, and counting the number of cells to which the third specific binding substance binds, in which all of the first, the second, and the third specific binding substances are specific binding substances that bind to a target cell surface protein, the occupancy rate is a proportion of cells to which the first specific binding substance binds, in cells expressing the target cell surface protein, the second specific binding substance is a specific binding substance that competes with the first specific binding substance, the third specific binding substance is a specific binding substance that binds to the target cell surface protein without competing with the first specific binding substance, and a value calculated by Expression (1) is the occupancy rate.

$$\text{Occupancy rate (\%) of the first specific binding substance} = (1 - (\text{Number of}$$

$$\text{cells to which the second specific binding substance binds/Number of cells to which the}$$

$$\text{third specific binding substance binds)}) \times 100 \cdots (1)$$

[2] The method according to [1], in which the bringing of the second specific binding substance into contact and the bringing of the third specific binding substance into contact are performed simultaneously.

[3] The method according to [1] or [2], in which the counting of the number of cells to which the second specific binding substance binds and the counting of the number of cells to which the third specific binding substance binds are performed simultaneously.

[4] The method according to any one of [1] to [3], in which the first specific binding substance is an antibody.

[5] The method according to [4], in which the target cell surface protein is human programmed death-1 (PD-1) and the first specific binding substance is an anti-human PD-1 antibody.

[6] The method according to [5], in which the first specific binding substance is nivolumab or pembrolizumab, the second specific binding substance is an anti-human PD-1 monoclonal antibody (clone EH12.2H7), and the third specific binding substance is an anti-human PD-1 monoclonal antibody (clone MIH4).

[7] A kit for measuring an occupancy rate of a first specific binding substance in a cell population, the kit including a second specific binding substance and a third specific binding substance, in which all of the first, the second, and the third specific binding substances are specific binding substances that bind to a target cell surface protein, the occupancy rate is a proportion of cells to which the first specific binding substance binds, in cells expressing the target cell surface protein, the second specific binding substance is a specific binding substance that competes with the first specific binding substance, and the third specific binding substance is a specific binding substance that binds to the target cell surface protein without competing with the first specific binding substance.

[8] The kit according to [7], in which the first specific binding substance is an antibody.

[9] The kit according to [8], in which the target cell surface protein is human PD-1 and the first specific binding substance is an anti-human PD-1 antibody.

[10] The kit according to [9], in which the first specific binding substance is nivolumab or pembrolizumab, the second specific binding substance is an anti-human PD-1 monoclonal antibody (clone EH12.2H7), and the third specific binding substance is an anti-human PD-1 monoclonal antibody (clone MIH4).

[11] A method for obtaining data for optimizing an administration interval or a dosage of an antibody drug to a patient, including bringing a second specific binding substance into contact with cells derived from the patient administered with the antibody drug, bringing a third specific binding substance into contact with the cells derived from the patient administered with the antibody drug, counting the number of cells to which the second specific binding substance binds, and counting the number of cells to which the third specific binding substance binds, in which both the second and the third specific binding substances are specific binding substances that bind to a target protein of the antibody drug, the second specific binding substance is a specific binding substance that competes with the antibody drug, the third specific binding substance is a specific binding substance that binds to the target protein of the antibody drug without competing with the antibody drug, and an occupancy rate of the antibody drug calculated by Expression (2) is the data for optimizing the administration interval or the dosage of the antibody drug to the patient.

$$\text{Occupancy rate (\%) of the antibody drug} = (1 - (\text{Number of cells to which the}$$

$$\text{second specific binding substance binds/Number of cells to which the third specific}$$

$$\text{binding substance binds)}) \times 100 \cdots (2)$$

[12] The method according to [11], in which the bringing of the second specific binding substance into contact and the bringing of the third specific binding substance into contact are performed simultaneously.

[13] The method according to [11] or [12], in which the counting of the number of cells to which the second specific binding substance binds and the counting of the number of cells to which the third specific binding substance binds are performed simultaneously.

[14] The method according to any one of [11] to [13], wherein the occupancy rate being higher than a reference value indicates the administration interval of the antibody drug to the patient to be lengthened or the dosage to be reduced, and the occupancy rate being lower than the reference value indicates the administration interval of the antibody drug to the patient to be shortened or the dosage to be increased.

[15] A method for obtaining data for optimizing an administration interval or a dosage of nivolumab or pembrolizumab to a patient, including bringing a second specific binding substance into contact with cells derived from a patient administered with nivolumab or pembrolizumab, bringing a third specific binding substance into contact with the cells derived from the patient administered with nivolumab or pembrolizumab, counting the number of cells to which the second specific binding substance binds; and counting the number of cells to which the third specific binding substance binds, in which both the second and the third specific binding substances are specific binding substances that bind to human PD-1, the second specific binding substance is a specific binding substance that competes with nivolumab or pembrolizumab, the third specific binding substance is a specific binding substance that binds to human PD-1 without competing with nivolumab or pembrolizumab, and an occupancy rate of nivolumab or pembrolizumab calculated by Expression (3) is the data for optimizing the administration interval or the dosage of nivolumab or pembrolizumab to the patient.

$$\text{Occupancy rate (\%) of nivolumab or pembrolizumab} = (1 - (\text{Number of cells to}$$

$$\text{which the second specific binding substance binds/Number of cells to which the third}$$

$$\text{specific binding substance binds)}) \times 100 \cdots (3)$$

[16] The method according to [15], in which the bringing of the second specific binding substance into contact and the bringing of the third specific binding substance into contact are performed simultaneously.

[17] The method according to [15] or [16], in which the counting of the number of cells to which the second specific binding substance binds and the counting of the number of cells to which the third specific binding substance binds are performed simultaneously.

[18] The method according to any one of [15] to [17], wherein the occupancy rate being higher than a reference value indicates the administration interval of nivolumab or pembrolizumab to the patient to be lengthened or the dosage to be reduced, and the occupancy rate being lower than the reference value indicates the administration interval of nivolumab or pembrolizumab to the patient to be shortened or the dosage to be increased.

[19] The method according to any one of [15] to [18], in which the second specific binding substance is an anti-human PD-1 monoclonal antibody (clone EH12.2H7), and the third specific binding substance is an anti-human PD-1 monoclonal antibody (clone MIH4).

Advantageous Effects of Invention

According to the present invention, a technique for simply measuring the dynamics of a specific binding substance can be provided.

BRIEF DESCRIPTION OF DRAWINGS

FIG. 1 is a graph showing the results obtained by analyzing the reactivity of an anti-PD-1 antibody (clone EH12.2H7) in Experimental Example 1.

FIG. 2 is a graph showing the results obtained by analyzing the reactivity of an anti-PD-1 antibody (clone MIH4) in Experimental Example 1.

FIG. 3 is a graph showing the results obtained by calculating the competition rate of an anti-PD-1 antibody (clone EH12.2H7 or clone MIH4) with nivolumab in Experimental Example 1.

FIG. 4 is a graph showing the results obtained by evaluating the reactivity of an anti-PD-1 antibody (clone EH12.2H7) after pretreatment with various concentrations of an anti-PD-1 antibody (clone MIH4) in Experimental Example 2.

FIG. 5 is a graph showing the results obtained by evaluating the reactivity of an anti-PD-1 antibody (clone MIH4) after pretreatment with various concentrations of an anti-PD-1 antibody (clone EH12.2H7) in Experimental Example 3.

FIGS 6(A) to 6(E) are graphs showing the results obtained by measuring the occupancy rate of nivolumab in a MIT9 cell line in Experimental Example 4.

FIG. 7 is a graph showing an occupancy rate of nivolumab measured in Experimental Example 4 and an actual occupancy rate of nivolumab.

FIGS. 8(A) to 8(E) are graphs showing the results obtained by measuring the binding of an PD-1 antibody (clone EH12.2H7) to T cells derived from a healthy person, which were prepared so that the occupancy rate of nivolumab was 0%, 25%, 50%, 75%, or 100% in Experimental Example 5.

FIG. 9 is a graph showing an occupancy rate of nivolumab measured in Experimental Example 5 and an actual occupancy rate of nivolumab.

FIGS. 10(A) to 10(C) are graphs showing the results obtained by measuring the occupancy rate of nivolumab in T cells derived from the body cavity fluid of a patient administered with nivolumab in Experimental Example 6.

FIGS. 11(A) to 11(E) are graphs showing the results obtained by measuring the occupancy rate of pembrolizumab in a MIT9 cell line in Experimental Example 7.

FIG. 12 is a graph showing an occupancy rate of pembrolizumab measured in Experimental Example 7 and an actual occupancy rate of pembrolizumab.

FIGS. 13(A) to 13(D) are graphs showing the results obtained by measuring the occupancy rate of nivolumab in peripheral blood T cells of a patient not administered with nivolumab in Experimental Example 8.

FIGS. 14(A) and 14(B) are graphs showing the results obtained by measuring the occupancy rate of nivolumab in peripheral blood T cells of a renal carcinoma patient administered with nivolumab in Experimental Example 9.

FIGS. 15(A) and 15(B) are graphs showing the results obtained by measuring the occupancy rate of pembrolizumab in peripheral blood T cells of a lung cancer patient administered with pembrolizumab in Experimental Example 10.

BEST MODE FOR CARRYING OUT THE INVENTION

[Method for measuring occupancy rate of specific binding substance in cell population]

In one embodiment, the present invention provides a method for measuring an occupancy rate of a first specific binding substance in a cell population, including a process of bringing a second specific binding substance into contact with the cell population, a process of bringing a third specific binding substance into contact with the cell population, a process of counting the number of cells to which the second specific binding substance binds, and a process of counting the number of cells to which the third specific binding substance binds, in which all of the first, the second, and the third specific binding substances are specific binding substances that bind to a target cell surface protein, the occupancy rate is a proportion of cells to which the first specific binding substance binds, in cells expressing the target cell surface protein, the second specific binding substance is a specific binding substance that competes with the first specific binding substance, the third specific binding substance is a specific binding substance that binds to the target cell surface protein without competing with the first specific binding substance, and a value calculated by Expression (1) is the occupancy rate.

$$\text{Occupancy rate (\%) of the first specific binding substance} = (1 - (\text{Number of}$$
$$\text{cells to which the second specific binding substance binds/Number of cells to which the}$$
$$\text{third specific binding substance binds})) \times 100 \cdots (1)$$

As will be described later in Examples, the inventors have clarified that the dynamics of a specific binding substance can be easily measured by the method of the present embodiment.

(Specific binding substance)

In the present specification, a specific binding substance means a substance that specifically binds to a target substance, and examples thereof include an antibody, an antibody fragment, and an aptamer. Examples of the target substance include a cell surface protein and the like. The specific binding substance may be a medicinal drug administered to a human or non-human animal.

The antibody may be prepared by immunizing an animal such as a mouse or may be prepared by screening an antibody library such as a phage library. As the antibody fragments, $F(ab')_2$, Fab', Fab, Fv, scFv, and the like are mentioned.

In a case where the antibody or the antibody fragment is a human antibody or a fragment thereof, side effects such as anaphylactic shock can be suppressed because the immunogenicity is low even when administered to humans. As the human antibodies, a chimeric antibody, a humanized antibody, a completely human antibody, and the like are mentioned. Here, the chimeric antibody means an antibody in which the variable region is derived from a non-human animal and at least part of the constant region is derived from a human. The humanized antibody means an antibody in which only the complementarity-determining region of the heavy chain and the light chain is derived from a non-human animal, and the constant region and the framework region are derived from a human. In addition, the completely human antibody means an antibody entirely derived from a human, including the complementarity determining region.

The aptamer is not particularly limited as long as it has a specific binding ability to a target substance, and includes a nucleic acid aptamer, a peptide aptamer, and the like.

(First specific binding substance)

In the method of the present embodiment, the first specific binding substance is a target of which the dynamics is to be measured, and is a specific binding substance that binds to a target cell surface protein. A specific example of the first specific binding substance includes an antibody. The antibody may be an antibody drug. The antibody drug may be, for example, an immune checkpoint inhibitor such as nivolumab or pembrolizumab, but is not limited thereto. Here, in a case where the first specific binding substance is nivolumab, pembrolizumab, or the like, the target cell surface protein is human PD-1. That is, the target cell surface protein is human PD-1 and the first specific binding substance may be an anti-human PD-1 antibody.

(Cell population)

As will be described later in Examples, the dynamics of the first specific binding substance can be easily measured by the method of the present embodiment. In the method of the present embodiment, the dynamics of the first specific binding substance is an occupancy rate of the first specific binding substance in a cell population. Here, the occupancy rate refers to the proportion (%) of cells to which the specific binding substance of interest (the first specific binding substance) binds, in cells that express the target cell surface protein.

In the method of the present embodiment, the cell population means a cell population in which the occupancy rate of the first specific binding substance is to be measured. The cell population is usually cells that have been brought into contact with the first specific binding substance. For example, in a case where the first specific binding substance is an antibody drug, the cell population includes a cell population derived from a patient administered with the antibody drug.

The cell population includes a cell population including cells to which the first specific binding substance binds, and is appropriately set according to a binding target of the first specific binding substance. Specific cell populations include, but are not limited to, peripheral blood lymphocytes, lymphocytes derived from pleural effusion fluid, lymphocytes derived from alveolar lavage fluid, and the like.

(Second specific binding substance)

The second specific binding substance is a specific binding substance that binds to the cell surface protein to which the first specific binding substance binds. In addition, the second specific binding substance competes with the first specific binding substance in binding to the target cell surface protein. That is, in a case where the first specific binding substance has bound to the target cell surface protein, the second specific binding substance is substantially impossible to bind to the target cell surface protein.

Here, "substantially impossible" means that even in a case where the first specific binding substance has bound to the target cell surface protein, a small number of cases where the second specific binding substance binds to the target cell surface protein by dissociating the first specific binding substance are allowed. However, in a case where the first specific binding substance has bound to the target cell surface protein, the second specific binding substance is preferably impossible to bind to the target cell surface protein.

In other words, it is preferable that an epitope of the second specific binding substance is the same as or adjacent to an epitope of the first specific binding substance, and a dissociation constant (Kd) of the first specific binding substance to the target cell surface protein is lower than a dissociation constant (Kd) of the second specific binding substance to the target cell surface protein. It is noted that the lower the dissociation constant, the higher the affinity.

(Third specific binding substance)

The third specific binding substance is a specific binding substance that binds to the cell surface protein to which the first specific binding substance binds. In addition, the third specific binding substance does not compete with the first specific binding substance in binding to the target cell surface protein. That is, even in a case where the first specific binding substance has bound to the target cell surface protein, the third specific binding substance is possible to bind to the target cell surface protein.

In other words, an epitope of the third specific binding substance is separated from an epitope of the first specific binding substance, and the binding of the third specific binding substance to the target cell surface protein substantially has no effect on the binding of the first specific binding substance to the target cell surface protein.

(Specific example of combination of first, second, and third specific binding substances)

For example, the first specific binding substance may be nivolumab or pembrolizumab, the second specific binding substance may be an anti-human PD-1 monoclonal antibody (clone EH12.2H7), and the third specific binding substance may be an anti-human PD-1 monoclonal antibody (clone MIH4). As will be described later in Examples, the dynamics of nivolumab or pembrolizumab can be easily measured by using a combination of these antibodies.

(Process of bringing second specific binding substance into contact)

In this process, the second specific binding substance is brought into contact with the cell population. As a result, in a case where the first specific binding substance has already bound to the target cell surface protein, the second specific binding substance does not bind to the target cell surface protein. In addition, in a case where the first specific binding substance has not bound to the target cell surface protein, the second specific binding substance bind to the target cell surface protein. As will be described later, the timing at which this process is performed may be appropriately changed.

(Process of bringing third specific binding substance into contact)

In this process, the third specific binding substance is brought into contact with the cell population. As a result, the third specific binding substance binds to the target cell surface protein whether or not the first specific binding substance has bound to the target cell surface protein. Accordingly, the third specific binding substance binds to all cells that express the target cell surface protein in the cell population. As will be described later, the timing at which this process is performed may be appropriately changed.

(Process of counting number of cells to which second specific binding substance binds)

In this process, the number of cells to which the second specific binding substance binds is counted. For example, it is convenient to count the number of cells to which the second specific binding substance binds by flow cytometry after labeling the second specific binding substance with a fluorescent dye. This process may be performed after the process of bringing the second specific binding substance into contact, and the timing at which this process is performed may be appropriately changed as will described later.

(Process of counting number of cells to which third specific binding substance binds)

In this process, the number of cells to which the third specific binding substance binds is counted. By counting the number of cells to which the third specific binding substance binds, the number of cells expressing the target cell surface protein can be counted. For example, it is convenient to count the number of cells to which the third specific binding substance binds by flow cytometry after labeling the third specific binding substance with a fluorescent dye. This process may be performed after the process of bringing the third specific binding substance into contact, and the timing at which this process is performed may be appropriately changed as will described later.

(Calculation of occupancy rate)

In this process, the occupancy rate of the first specific binding substance in the cell population is calculated. The occupancy rate is a value calculated by Expression (1).

$$\text{Occupancy rate (\%) of the first specific binding substance} = (1 - (\text{Number of cells to which the second specific binding substance binds/Number of cells to which the third specific binding substance binds})) \times 100 \cdots (1)$$

For example, an occupancy rate of 100% means that the first specific binding substance has bound to all cells that express the target cell surface protein in the cell population. In this case, since the first specific binding substance has bound to the target cell surface protein, the second specific binding substance does not bind to the target cell surface protein.

In addition, for example, an occupancy rate of 80% means that the first specific binding substance has bound to 80% of cells that express the target cell surface protein in the cell population. In this case, the remaining 20% of the cells that express the target cell surface protein have not bound to the first specific binding substance and may bind to the second specific binding substance.

(Order of implementation of each of above process)

Either the process of bringing the second specific binding substance into contact or the process of bringing the third specific binding substance into contact may be implemented first. For example, the process of bringing the third specific binding substance into contact may be implemented after the process of bringing the second specific binding substance into contact, or the order may be reversed. Alternatively, the process of bringing the second specific binding substance into contact and the process of bringing the third specific binding substance into contact may be implemented simultaneously. For example, the process of bringing the second specific binding substance into contact and the process of bringing the third specific binding substance into contact can be performed simultaneously by putting the cell population, the second specific binding substance, and the third specific binding substance into the same test tube to bring them into contact with each other.

In addition, either the process of counting the number of cells to which the second specific binding substance has bound or the process of counting the number of cells to which the third specific binding substance has bound may be implemented first. For example, the process of counting the number of cells to which the third specific binding substance has bound may be implemented after the process of counting the number of cells to which the second specific binding substance has bound, or the order may be reversed.

Alternatively, the process of counting the number of cells to which the second specific binding substance has bound or the process of counting the number of cells to which the third specific binding substance has bound may be implemented simultaneously. The process of counting the number of cells to which the second specific binding substance has bound and the process of counting the number of cells to which the third specific binding substance has bound can be implemented simultaneously, for example, by labeling each of the second specific binding substance and the third specific binding substance so as to be distinguishable and analyzing the cell population brought into contact with the second specific binding substance and the third specific binding substance by flow cytometry, or the like.

Alternatively, the process of bringing the second specific binding substance into contact, the process of counting the number of cells to which the second specific binding substance has bound, the process of bringing the third specific binding substance into contact, and the process of counting the number of cells to which the third specific binding substance has bound may be implemented in this order.

Alternatively, the process of bringing the third specific binding substance into contact, the process of counting the number of cells to which the third specific binding substance has bound, the process of bringing the second specific binding substance into contact, and the process of counting the number of cells to which the second specific binding substance has bound may be implemented in this order. In this case, after recovering the cells to which the third specific binding substance has bound by using a cell sorter or the like, the process of bringing the second specific binding substance into contact with the recovered cells and the process of counting the number of cells to which the second specific binding substance has bound may be implemented.

(Other specific binding substance)

Further, in addition to the second specific binding substance and the third specific binding substance, for example, a process of bringing other specific binding substance such as an anti-CD3 antibody into contact may be implemented, and an occupancy rate of the first specific binding substance in a cell population to which the other specific binding substance has bound may be measured. For example, the occupancy rate of the first specific binding substance may be calculated after gating the cell population to which the other specific binding substance has bound.

The other specific binding substance is not limited to the anti-CD3 antibody, and any specific binding substance to an antigen can be used. By reacting cells with the other specific binding substance, the expression of a specific antigen can be simultaneously analyzed together with measuring the occupancy rate of the first specific binding substance.

The method of the present embodiment can be applied to, for example, a method for obtaining data for optimizing an administration interval or a dosage of an antibody drug to a patient. Specifically, the method described above may be implemented using cells derived from a patient to which an antibody drug has been administered as the cell population.

In this case, the method is the same as the method described above except that the first specific binding substance is an antibody drug. As a result, it is possible to calculate the occupancy rate of the antibody drug in the cells derived from the patient. Then, based on the occupancy rate of the antibody drug in the cells derived from the patient, the administration interval or the dosage of the antibody drug to the patient can be optimized.

For example, in a case where the occupancy rate is higher than a reference value, the administration interval of the antibody drug to the patient may be lengthened or the dosage may be reduced. In addition, in a case where the occupancy rate is lower than the reference value, the administration interval of the antibody drug to the patient may be shortened or the dosage may be increased.

[Kit for measuring occupancy rate of first specific binding substance in cell population]

In one embodiment, the present invention provides a kit for measuring an occupancy rate of a first specific binding substance in a cell population, the kit including a second specific binding substance and a third specific binding substance, in which all of the first, the second, and the third specific binding substance are specific binding substances that bind to a target cell surface protein, the occupancy rate is a proportion of cells to which the first specific binding substance binds, in cells expressing the target cell surface protein, the second specific binding substance is a specific binding substance that competes with the first specific binding substance, and the third specific binding substance is a specific binding substance that binds to the target cell surface protein without competing with the first specific binding substance.

In the kit of the present embodiment, the cell population, the first, the second, and the third specific binding substances, and the target cell surface protein are the same as those described above. That is, a specific example of the first specific binding substance includes an antibody.

In addition, the target cell surface protein is human PD-1 and the first specific binding substance may be an anti-human PD-1 antibody.

Further, the first specific binding substance may be nivolumab or pembrolizumab, the second specific binding substance may be an anti-human PD-1 monoclonal antibody (clone EH12.2H7), and the third specific binding substance may be an anti-human PD-1 monoclonal antibody (clone MIH4). With such a kit, the dynamics of nivolumab or pembrolizumab in a cell population can be easily measured.

[Method for obtaining data for optimizing administration interval or dosage of antibody drug to patient]

In one embodiment, the present invention provides a method for obtaining data for optimizing an administration interval or a dosage of an antibody drug to a patient, including a process of bringing a second specific binding substance into contact with cells derived from the patient administered with the antibody drug, a process of bringing a third specific binding substance into contact with the cells derived from the patient administered with the antibody drug, a process of counting the number of cells to which the second specific binding substance binds, and a process of counting the number of cells to which the third specific binding substance binds, in which both the second and the third specific binding substances are specific binding substances that bind to a target protein of the antibody drug, the second specific binding substance is a specific binding substance that competes with the antibody drug, the third specific binding substance is a specific binding substance that binds to the target protein of the antibody drug without competing with the antibody drug, and an occupancy rate of the antibody drug calculated by Expression (2) is the data for optimizing the administration interval or the dosage of the antibody drug to the patient.

$$\text{Occupancy rate (\%) of the antibody drug} = (1 - (\text{Number of cells to which the second specific binding substance binds/Number of cells to which the third specific binding substance binds})) \times 100 \cdots (2)$$

According to the method of the present embodiment, the dynamics of an antibody drug can be easily measured, and data for optimizing an administration interval or a dosage of the antibody drug to a patient can be obtained. The method of the present embodiment does not include a process of making a judgment by a doctor.

In the method of the present embodiment, the optimization of an administration interval or a dosage of an antibody drug can be referred to as the individualization of the administration interval or the dosage of an antibody drug. That is, the method of the present embodiment can provide data for determining an optimal administration interval or dosage of an antibody drug according to an individual patient. As a result, for example, in a case where a patient develops a serious immunological adverse event, it is possible to predict how long the risk of adverse events will be prolonged. In addition, by enabling the use of a suitable amount of an antibody drug, it becomes possible to suppress the cost of an expensive antibody drug or the like.

(Cells derived from patient)

In the method of the present embodiment, as cells derived from a patient, suitable cells are appropriately selected according to the characteristics of an antibody drug. The cells derived from a patient include, but are not limited to, peripheral blood lymphocytes, lymphocytes derived from pleural effusion fluid, lymphocytes derived from alveolar lavage fluid, and the like.

(Second and third specific binding substances)

In the method of the present embodiment, the second and the third specific binding substances include the same as the second and the third specific binding substance in a case where the first specific binding substance in the embodiment of the above-described "a method for measuring an occupancy rate of a first specific binding substance in a cell population" is an antibody drug.

(Process of bringing second and third specific binding substance into contact and process of counting number of cells to which second and third specific binding substance have bound)

In the method of the present embodiment, either the process of bringing the second specific binding substance into contact or the process of bringing the third specific binding substance into contact may be performed first as in the case where the first specific binding substance in the embodiment of the above-described "a method for measuring an occupancy rate of a first specific binding substance in a cell population" is an antibody drug. For example, the process of bringing the third specific binding substance into contact may be implemented after the process of bringing the second specific binding substance into contact, or the order may be reversed. Alternatively, the process of bringing the second specific binding substance into contact and the process of bringing the third specific binding substance into contact may be implemented simultaneously. For example, the process of bringing the second specific binding substance into contact and the process of bringing the third specific binding substance into contact can be performed simultaneously by putting the cells derived from a patient, the second specific binding substance, and the third specific binding substances into the same test tube to bring them into contact with each other.

In addition, either the process of counting the number of cells to which the second specific binding substance has bound or the process of counting the number of cells to which the third specific binding substance has bound may be implemented first. For example, the process of counting the number of cells to which the third specific binding substance has bound may be implemented after the process of counting the number of cells to which the second specific binding substance has bound, or the order may be reversed.

Alternatively, the process of counting the number of cells to which the second specific binding substance has bound or the process of counting the number of cells to which the third specific binding substance has bound may be implemented simultaneously. The process of counting the number of cells to which the second specific binding substance has bound and the process of counting the number of cells to which the third specific binding substance has bound can be implemented simultaneously, for example, by labeling each of the second specific binding substance and the third specific binding substance so as to be distinguishable and analyzing the cells derived from a patient that have been brought into contact with the second specific binding substance and the third specific binding substance by flow cytometry, or the like.

Alternatively, the process of bringing the second specific binding substance into contact, the process of counting the number of cells to which the second specific binding substance has bound, the process of bringing the third specific binding substance into contact, and the process of counting the number of cells to which the third specific binding substance has bound may be implemented in this order.

Alternatively, the process of bringing the third specific binding substance into contact, the process of counting the number of cells to which the third specific binding substance has bound, the process of bringing the second specific binding substance into contact, and the process of counting the number of cells to which the second specific binding substance has bound may be implemented in this order. In this case, after recovering the cells to which the third specific binding substance has bound by using a cell sorter or the like, the process of bringing the second specific binding substance into contact with the recovered cells and the process of counting the number of cells to which the second specific binding substance has bound may be implemented.

(Other specific binding substance)

Further, in addition to the second specific binding substance and the third specific binding substance, for example, a process of bringing other specific binding substance such as an anti-CD3 antibody into contact may be implemented, and an occupancy rate of an antibody drug in a cell population to which the other specific binding substance has bound may be measured. For example, the occupancy rate of the antibody drug may be calculated after gating the cell population to which the other specific binding substance has bound.

The other specific binding substance is not limited to the anti-CD3 antibody, and any specific binding substance to an antigen can be used. By reacting cells with the other specific binding substance, the expression of a specific antigen can be simultaneously analyzed together with measuring the occupancy rate of the antibody drug.

(Data for optimizing administration interval or dosage of antibody drug to patient)

In the method of the present embodiment, the occupancy rate of the antibody drug is the data for optimizing an administration interval or a dosage of an antibody drug to a patient. The occupancy rate being higher than a reference value indicates that the administration interval of the antibody drug to the patient should be lengthened or the dosage should be reduced.

Here, the reference value of the occupancy rate is, for example, an optimal value of the occupancy rate set in advance. The optimal value of the occupancy rate is, for example, an occupancy rate that maximizes the therapeutic effects to disease by the antibody drug and minimizes side effects such as immunological adverse events.

In a case where it is indicated that the occupancy rate of the antibody drug is higher than the reference value by the method of the present embodiment, it means that the dosage of the antibody drug is sufficient or excessive. In such a case, the occupancy rate of the antibody drug in the patient's cells can be set to be close to the reference value by lengthening the administration interval of the antibody drug to the patient or reducing the dosage of the antibody drug to the patient.

Alternatively, in a case where it is indicated that the occupancy rate of the antibody drug is lower than the reference value by the method of the present embodiment, it means that the dosage of the antibody drug is insufficient. In such a case, the occupancy rate of the antibody drug in the patient's cells can be set to be close to the reference value by shortening the administration interval of the antibody drug to the patient or increasing the dosage of the antibody drug to the patient.

[Method for obtaining data for optimizing administration interval or dosage of nivolumab or pembrolizumab to patient]

In one embodiment, the present invention provides a method for obtaining data for optimizing an administration interval or a dosage of nivolumab or pembrolizumab to a patient, including a process of bringing a second specific binding substance into contact with cells derived from a patient administered with nivolumab or pembrolizumab, a process of bringing a third specific binding substance into contact with the cells derived from the patient administered with nivolumab or pembrolizumab, a process of counting the number of cells to which the second specific binding substance binds, and a process of counting the number of cells to which the third specific binding substance binds, in which both the second and the third specific binding substances are specific binding substances that bind to human PD-1, the second specific binding substance is a specific binding substance that competes with nivolumab or pembrolizumab, the third specific binding substance is a specific binding substance that binds to human PD-1 without competing with nivolumab or pembrolizumab, and an occupancy rate of nivolumab or pembrolizumab calculated by Expression (3) is the data for optimizing the administration interval or the dosage of nivolumab or pembrolizumab to the patient.

$$\text{Occupancy rate (\%) of nivolumab or pembrolizumab} = (1 - (\text{Number of cells to which the second specific binding substance binds/Number of cells to which the third specific binding substance binds})) \times 100 \cdots (3)$$

According to the method of the present embodiment, the dynamics of nivolumab or pembrolizumab can be easily measured, and data for optimizing an administration interval or a dosage of nivolumab or pembrolizumab to a patient can be obtained. The method of the present embodiment does not include a process of making a judgment by a doctor.

In the method of the present embodiment, the optimization of the administration interval or the dosage of nivolumab or pembrolizumab can be referred to as the individualization of the administration interval or the dosage of nivolumab or pembrolizumab. That is, the method of the present embodiment can provide data for determining an optimal administration interval or dosage of nivolumab or pembrolizumab according to an individual patient. As a result, for example, in a case where a patient develops a serious immunological adverse event, it is possible to predict how long the risk of adverse events will be prolonged. In addition, by enabling the use of a suitable amount of nivolumab or pembrolizumab, it becomes possible to suppress the cost of an expensive antibody drug or the like.

(Cells derived from patient)

In the method of the present embodiment, the cells derived from a patient include, for example, peripheral blood lymphocytes, lymphocytes derived from pleural effusion fluid, lymphocytes derived from alveolar lavage fluid, and the like.

(Second and third specific binding substances)

In the method of the present embodiment, the second and the third specific binding substances include the same as the second and the third specific binding substances in a case where the first specific binding substance in the embodiment of the above-described "a method for measuring an occupancy rate of a first specific binding substance in a cell population" is nivolumab or pembrolizumab.

As a more specific example, an anti-human PD-1 monoclonal antibody (clone EH12.2H7) is mentioned as the second specific binding substance. In addition, an anti-human PD-1 monoclonal antibody (clone MIH4) is mentioned as the third specific binding substance. As will be described later in Examples, the inventors have revealed that the occupancy rate of nivolumab or pembrolizumab can be measured by using these antibodies.

(Process of bringing second and third specific binding substance into contact and process of counting number of cells to which second and third specific binding substance have bound)

In the method of the present embodiment, either the process of bringing the second specific binding substance into contact or the process of bringing the third specific binding substance into contact may be performed first as in the case where the first specific binding substance in the embodiment of the above-described "a method for measuring an occupancy rate of a first specific binding substance in a cell population" is nivolumab or pembrolizumab. For example, the process of bringing the third specific binding substance into contact may be implemented after the process of bringing the second specific binding substance into contact, or the order may be reversed. Alternatively, the process of bringing the second specific binding substance into contact and the process of bringing the third specific binding substance into contact may be implemented simultaneously. For example, the process of bringing the second specific binding substance into contact and the process of bringing the third specific binding substance into contact can be performed simultaneously by putting the cells derived from a patient, the second specific binding substance, and the third specific binding substances into the same test tube to bring them into contact with each other.

In addition, either the process of counting the number of cells to which the second specific binding substance has bound or the process of counting the number of cells to which the third specific binding substance has bound may be implemented first. For example, the process of counting the number of cells to which the third specific binding substance has bound may be implemented after the process of counting the number of cells to which the second specific binding substance has bound, or the order may be reversed.

Alternatively, the process of counting the number of cells to which the second specific binding substance has bound or the process of counting the number of cells to which the third specific binding substance has bound may be implemented simultaneously. The process of counting the number of cells to which the second specific binding substance has bound and the process of counting the number of cells to which the third specific binding substance has bound can be implemented simultaneously, for example, by labeling each of the second specific binding substance and the third specific binding substance so as to be distinguishable and analyzing the cells derived from a patient that have been brought into contact with the second specific binding substance and the third specific binding substance by flow cytometry, or the like.

Alternatively, the process of bringing the second specific binding substance into contact, the process of counting the number of cells to which the second specific binding substance has bound, the process of bringing the third specific binding substance into contact, and the process of counting the number of cells to which the third specific binding substance has bound may be implemented in this order.

Alternatively, the process of bringing the third specific binding substance into contact, the process of counting the number of cells to which the third specific binding substance has bound, the process of bringing the second specific binding substance into contact, and the process of counting the number of cells to which the second specific binding substance has bound may be implemented in this order. In this case, after recovering the cells to which the third specific binding substance has bound by using a cell sorter or the like, the process of bringing the second specific binding substance into contact with the recovered cells and the process of counting the number of cells to which the second specific binding substance has bound may be implemented.

(Other specific binding substance)

Further, in addition to the second specific binding substance and the third specific binding substance, for example, a process of bringing other specific binding substance such as an anti-CD3 antibody into contact may be implemented, and an occupancy rate of an antibody drug in a cell population to which the other specific binding substance has bound may be measured. For example, the occupancy rate of nivolumab or pembrolizumab may be calculated after gating the cell population to which the other specific binding substance has bound.

The other specific binding substance is not limited to the anti-CD3 antibody, and any specific binding substance to an antigen can be used. By reacting cells with the other specific binding substance, the expression of a specific antigen can be simultaneously analyzed together with measuring the occupancy rate of nivolumab or pembrolizumab.

(Data for optimizing administration interval or dosage of nivolumab or pembrolizumab to patient)

In the method of the present embodiment, an occupancy rate of nivolumab or pembrolizumab is data for optimizing an administration interval or a dosage of nivolumab or pembrolizumab to a patient. The occupancy rate being higher than a reference value indicates that the administration interval of nivolumab or pembrolizumab to the patient should be lengthened or the dosage should be reduced.

Here, the reference value of the occupancy rate is, for example, an optimal value of the occupancy rate set in advance. The optimal value of the occupancy rate is, for example, an occupancy rate that maximizes the therapeutic effects to disease by nivolumab or pembrolizumab and minimizes side effects such as immunological adverse events. The optimal value of the occupancy rate of nivolumab or pembrolizumab can be appropriately set according to the patient's condition or the like and may be for example 90%, for example 95%, and for example 98%.

In a case where it is indicated that the occupancy rate of nivolumab or pembrolizumab is higher than the reference value by the method of the present embodiment, the dosage of nivolumab or pembrolizumab is sufficient or excessive. In such a case, the occupancy rate of nivolumab or pembrolizumab in the patient's cells can be made close to the reference value by lengthening the administration interval of nivolumab or pembrolizumab to the patient or reducing the dosage of nivolumab or pembrolizumab to the patient.

Alternatively, in a case where it is indicated that the occupancy rate of nivolumab or pembrolizumab is lower than the reference value by the method of the present embodiment, it means that the dosage of nivolumab or pembrolizumab is insufficient. In such a case, the occupancy rate of the antibody drug in the patient's cells can be set to be close to the reference value by shortening the administration interval of the antibody drug to the patient or increasing the dosage of the antibody drug to the patient.

[Other Embodiments]

In one embodiment, the present invention provides a method for optimizing an administration interval or a dosage of an antibody drug to a patient, including bringing a second specific binding substance into contact with cells derived from the patient administered with the antibody drug, bringing a third specific binding substance into contact with the cells derived from the patient administered with the antibody drug, counting the number of cells to which the second specific binding substance binds, and counting the number of cells to which the third specific binding substance binds, in which both the second and the third specific binding substances are specific binding substances that bind to a target protein of the antibody drug, the second specific binding substance is a specific binding substance that competes with the antibody drug, the third specific binding substance is a specific binding substance that binds to the target protein of the antibody drug without competing with the antibody drug, the administration interval of the antibody drug to the patient is lengthened in a case where an occupancy rate of the antibody drug calculated by Expression (2) is higher than a reference value, and the administration interval of the antibody drug to the patient is shortened in a case where the occupancy rate of the antibody drug is lower than the reference value.

$$\text{Occupancy rate (\%) of the antibody drug} = (1 - (\text{Number of cells to which the}$$

$$\text{second specific binding substance binds/Number of cells to which the third specific}$$

$$\text{binding substance binds})) \times 100 \cdots (2)$$

In the method of the present embodiment, the bringing of the second specific binding substance into contact and the bringing of the third specific binding substance into contact may be performed simultaneously. In addition, the counting of the number of cells to which the second specific binding substance binds and the counting of the number of cells to which the third specific binding substance binds may be performed simultaneously.

In one embodiment, the present invention provides a method for optimizing an administration interval or a dosage of nivolumab or pembrolizumab to a patient, including bringing a second specific binding substance into contact with cells derived from a patient administered with nivolumab or pembrolizumab, bringing a third specific binding substance into contact with the cells derived from the patient administered with nivolumab or pembrolizumab, counting the number of cells to which the second specific binding substance binds, and counting the number of cells to which the third specific binding substance binds, in which both the second and the third specific binding substances are specific binding substances that bind to human PD-1, the second specific binding substance is a specific binding substance that competes with nivolumab or pembrolizumab, the third specific binding substance is a specific binding substance that binds to human PD-1 without competing with nivolumab or pembrolizumab, the administration interval of nivolumab or pembrolizumab to the patient is lengthened in a case where an occupancy rate of nivolumab or pembrolizumab calculated by Expression (3) is higher than a reference value, and the administration interval of nivolumab or pembrolizumab to the patient is shortened in a case where the occupancy rate of nivolumab or pembrolizumab is lower than the reference value.

$$\text{Occupancy rate (\%) of nivolumab or pembrolizumab} = (1 - (\text{Number of cells to}$$

$$\text{which the second specific binding substance binds/Number of cells to which the third}$$

$$\text{specific binding substance binds)}) \times 100 \cdots (3)$$

In the method of the present embodiment, the bringing of the second specific binding substance into contact and the bringing of the third specific binding substance into contact may be performed simultaneously. In addition, the counting of the number of cells to which the second specific binding substance binds and the counting of the number of cells to which the third specific binding substance binds may be performed simultaneously.

EXAMPLES

Next, the present invention will be described in more detail by showing Examples, but the present invention is not limited to following Examples.

[Experimental Example 1]

(Evaluation 1 of anti-PD-1 antibody)

<< Clone EH12.2H7 >>

The reactivity of an anti-PD-1 antibody (clone EH12.2H7, BioLegend Inc.) was evaluated using MIT9 cells, a cell line derived from mouse fibroblasts, in which human PD-1 was forcibly expressed.

First, $2 \times 10^5$ cells of the MIT9 cell line were dispensed into tubes. Subsequently, nivolumab which is an anti-PD-1 antibody drug was added to each of the dispensed cells at 0, 0.11, 0.33, 1, 3.3, and 10 $\mu$g/mL, and reacted at 4°C for 30 minutes.

Subsequently, the individual cells were washed twice with 600 $\mu$L of phosphate buffer (PBS). Subsequently, 1 $\mu$g/mL of the PE-labeled anti-PD-1 antibody (clone EH12.2H7, BioLegend Inc.) was added to the individual cells and reacted at 4°C for 20 minutes.

Subsequently, the individual cells were washed twice with 600 $\mu$L of PBS. Subsequently, the binding of the PE-labeled anti-PD-1 antibody (clone EH12.2H7) to the individual cells were analyzed using a flow cytometer (model "FACSVerse", BD Bioscience).

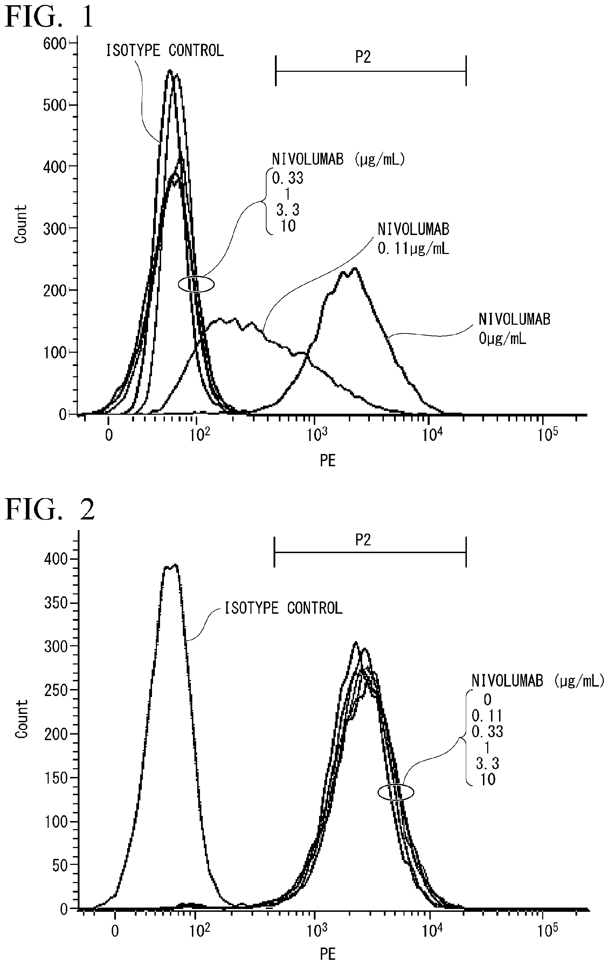

FIG. 1 is a graph showing the analysis results. As a result, it was revealed that as the concentration of nivolumab initially reacted increases, the amount of the PE-labeled anti-PD-1 antibody (clone EH12.2H7) bound to MIT9 cells decreases.

This result revealed that the anti-PD-1 antibody (clone EH12.2H7) competed with nivolumab.

<< Clone MIH4 >>

The reactivity of an anti-PD-1 antibody (Clone MIH4) was evaluated in the same manner as described above except that a PE-labeled anti-PD-1 antibody (clone MIH4, eBioscience Inc.) was used instead of the PE-labeled anti-PD-1 antibody (clone EH12.2H7, BioLegend Inc.).

First, $2 \times 10^5$ cells of the MIT9 cell line were dispensed into tubes. Subsequently, nivolumab which is an anti-PD-1 antibody drug was added to each of the dispensed cells at 0, 0.11, 0.33, 1, 3.3, and 10 $\mu$g/mL, and reacted at 4°C for 30 minutes.

Subsequently, the individual cells were washed twice with 600 $\mu$L of PBS. Subsequently, 2 $\mu$g/mL of the PE-labeled anti-PD-1 antibody (clone MIH4, eBioscience Inc.) was added to the individual cells and reacted at 4°C for 20 minutes.

Subsequently, the individual cells were washed twice with 600 $\mu$L of PBS. Subsequently, the binding of the PE-labeled anti-PD-1 antibody (clone MIH4) to the individual cells were analyzed using the flow cytometer (model "FACSVerse", BD Bioscience).

FIG. 2 is a graph showing the analysis results. As a result, it was revealed that the PE-labeled anti-PD-1 antibody (clone MIH4) binds to MIT9 cells regardless of the concentration of nivolumab initially reacted.

This result revealed that the anti-PD-1 antibody (clone MIH4) binds to PD-1 without competing with nivolumab.

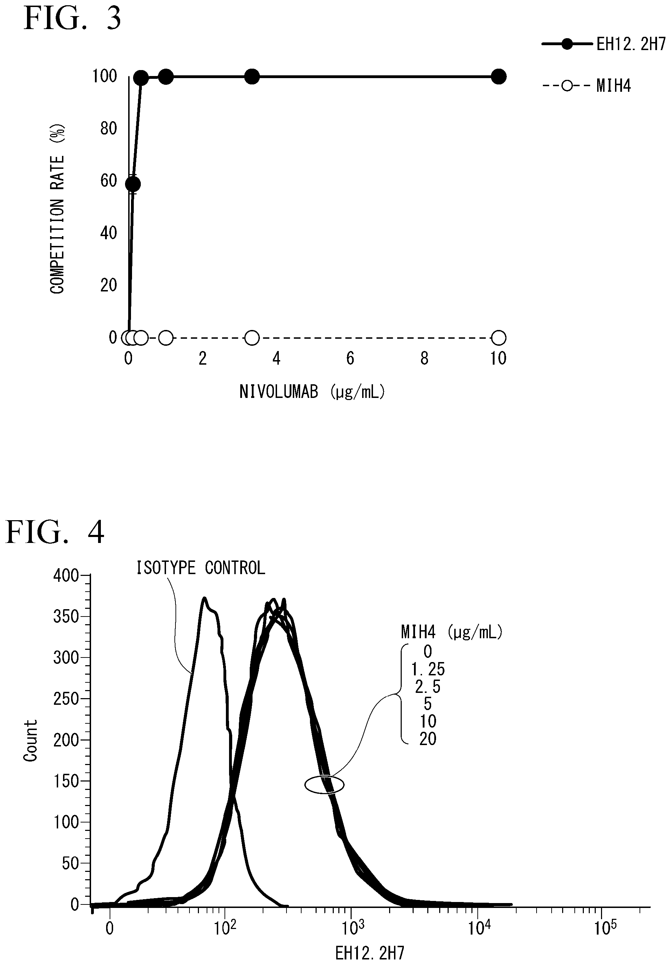

FIG. 3 is a graph showing the results obtained by calculating the competition rate of an anti-PD-1 antibody (clone EH12.2H7 or clone MIH4) with nivolumab based on the analysis results shown in FIG. 1 and FIG. 2. The competition rate was calculated by following Expression (4).

Competition rate (%) = (Total number of cells - Number of anti-PD-1 antibody (clone EH12.2H7 or clone MIH4) positive cells)/Total number of cells $\times$ 100···(4)

[Experimental Example 2]

(Evaluation 2 of anti-PD-1 antibody)

The competition between the anti-PD-1 antibody (clone EH12.2H7) and the anti-PD-1 antibody (clone MIH4) was evaluated.

First, $2 \times 10^5$ cells of the MIT9 cell line were dispensed into tubes. Subsequently, 0, 1.25, 2.5, 5, 10, and 20 $\mu$g/mL of the PE-labeled anti-PD-1 antibody (clone MIH4, eBioscience Inc.) was added to each of the dispensed cells and reacted at 4°C for 20 minutes.

Subsequently, the individual cells were washed twice with 600 $\mu$L of PBS. Subsequently, 4 $\mu$g/mL of an Alexa Fluor 488-labeled anti-PD-1 antibody (clone EH12.2H7, BioLegend Inc.) was added to the individual cells and reacted at 4°C for 20 minutes.

Subsequently, the individual cells were washed twice with 600 $\mu$L of PBS. Subsequently, the binding of the Alexa Fluor 488-labeled anti-PD-1 antibody (clone EH12.2H7) to the individual cells were analyzed using a flow cytometer (model "FACSVerse", BD Bioscience).

FIG. 4 is a graph showing the analysis results. As a result, it was revealed that the Alexa Fluor 488-labeled anti-PD-1 antibody (clone EH12.2H7) binds to MIT9 cells regardless of the concentration of the PE-labeled anti-PD-1 antibody (clone MIH4) initially reacted.

This result revealed that the anti-PD-1 antibody (clone EH12.2H7) binds to PD-1 without competing with the anti-PD-1 antibody (clone MIH4).

[Experimental Example 3]

(Evaluation 3 of anti-PD-1 antibody)

The competition between the anti-PD-1 antibody (clone EH12.2H7) and the anti-PD-1 antibody (clone MIH4) was evaluated by reversing the order of reactions in which the antibodies were reacted in Experimental Example 2.

First, $2 \times 10^5$ cells of the MIT9 cell line were dispensed into tubes. Subsequently, 0, 0.5, 1, 2, 4, and 8 $\mu$g/mL of the Alexa Fluor 488-labeled anti-PD-1 antibody (clone EH12.2H7, BioLegend Inc.) was added to each of the dispensed cells and reacted at 4°C for 20 minutes.

Subsequently, the individual cells were washed twice with 600 $\mu$L of PBS. Subsequently, 10 $\mu$g/mL of the PE-labeled anti-PD-1 antibody (clone MIH4, eBioscience Inc.) was added to the individual cells and reacted at 4°C for 20 minutes.

Subsequently, the individual cells were washed twice with 600 $\mu$L of PBS. Subsequently, the binding of the PE-labeled anti-PD-1 antibody (clone MIH4) to the individual cells were analyzed using the flow cytometer (model "FACSVerse", BD Bioscience).

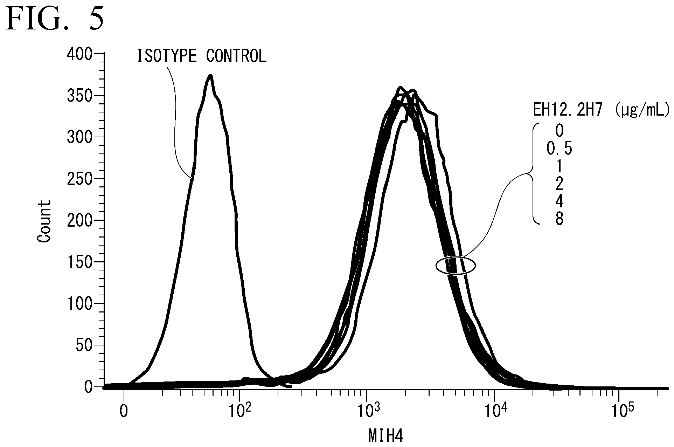

FIG. 5 is a graph showing the analysis results. As a result, it was revealed that the PE-labeled anti-PD-1 antibody (clone MIH4) binds to MIT9 cells regardless of the concentration of the Alexa Fluor 488-labeled anti-PD-1 antibody (clone EH12.2H7) initially reacted.

This result further supports that the anti-PD-1 antibody (clone EH12.2H7) binds to PD-1 without competing with the anti-PD-1 antibody (clone MIH4).

[Experimental Example 4]

(Measurement 1 of occupancy rate of nivolumab)

The anti-PD-1 antibody (clone EH12.2H7) and the anti-PD-1 antibody (clone MIH4) were used to measure an occupancy rate of nivolumab in the MIT9 cell line.

First, 10 $\mu$g/mL of nivolumab was added to the MIT9 cell line and reacted at 4°C for 30 minutes. Subsequently, cells were washed three times with 600 $\mu$L of PBS. Subsequently, a MIT9 cell line not reacted with nivolumab was mixed with the MIT9 cell line reacted with nivolumab so that the proportion of the MIT9 cell line reacted with nivolumab was 0%, 25%, 50%, 75%, and 100%, and then $2 \times 10^5$ cells of the mixed cell lines were dispensed into tubes. As a result, MIT9 cell lines each having occupancy rates of nivolumab of 0%, 25%, 50%, 75%, and 100% were obtained.

Subsequently, 4 $\mu$g/mL of the Alexa Fluor 488-labeled anti-PD-1 antibody (clone EH12.2H7, Biolegend Inc.) and 10 $\mu$g/mL of the PE-labeled anti-PD-1 antibody (clone MIH4, eBioscience Inc.) were added to the individual cells and reacted at 4°C for 20 minutes.

Subsequently, the individual cells were washed twice with 600 $\mu$L of PBS. Subsequently, the binding of the Alexa Fluor 488-labeled anti-PD-1 antibody (clone EH12.2H7) and the PE-labeled anti-PD-1 antibody (clone MIH4) to the individual cells were analyzed using the flow cytometer (model "FACSVerse", BD Bioscience).

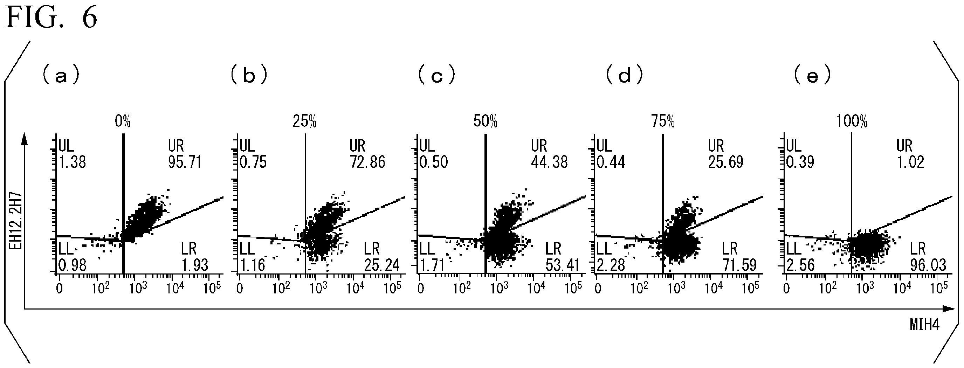

FIGS. 6(A) to 6(E) are graphs showing the analysis results. FIGS. 6(A) to 6(E) show the results obtained in the MIT9 cell lines prepared so that the occupancy rates of nivolumab were respectively 0%, 25%, 50%, 75%, and 100%. In FIGS. 6(A) to 6(E), the vertical axis represents the fluorescence intensity of Alexa Fluor 488, and the horizontal axis represents the fluorescence intensity of PE.

From the results, it was recognized that the fluorescence intensity of Alexa Fluor 488 tended to decrease as the occupancy rate of nivolumab increased. On the other hand, it was recognized that the fluorescence intensity of PE tended not to change even in a case where the occupancy rate of nivolumab changed.

Subsequently, based on the results of FIGS. 6(A) to 6(E), the occupancy rate of nivolumab was calculated by following Expression (3A).

$$\text{Occupancy rate of nivolumab } (\%) = (1 - (\text{Proportion of cells to which anti-PD-1}$$

$$\text{antibody (EH12.2H7) has bound/Proportion of cells to which anti-PD-1 antibody (clone}$$

$$\text{MIH4)) has bound}) \times 100 \cdots (3A)$$

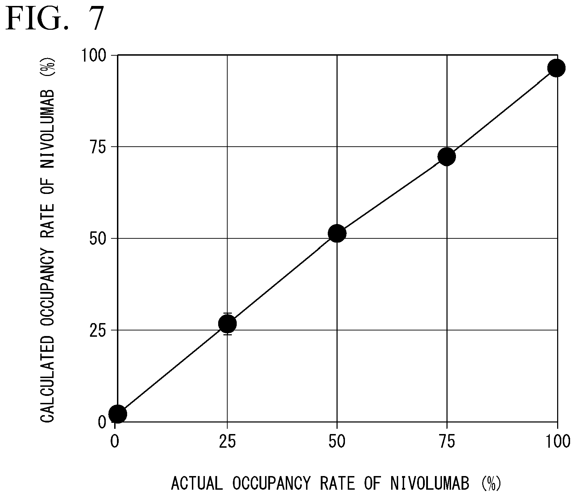

FIG. 7 is a graph showing an occupancy rate of nivolumab calculated by above Expression (3A) and an actual occupancy rate of nivolumab. From the result, it was confirmed that the occupancy rate of nivolumab calculated by above Expression (3A) well matched the actual occupancy rate of nivolumab. This result indicates that the occupancy rate of nivolumab can be measured by the method of this Experimental Example.

[Experimental Example 5]

(Measurement 2 of occupancy rate of nivolumab)

The anti-PD-1 antibody (clone EH12.2H7) and the anti-PD-1 antibody (clone MIH4) were used to measure an occupancy rate of nivolumab in T cells in peripheral blood lymphocytes of a healthy person.

First, 10 $\mu$g/mL of nivolumab was added to the peripheral blood lymphocytes and reacted at 4°C for 30 minutes. Subsequently, peripheral blood lymphocytes not reacted with nivolumab was mixed with the peripheral blood lymphocytes reacted with nivolumab so that the proportion of the peripheral blood lymphocytes reacted with nivolumab were 0%, 25%, 50%, 75%, and 100%, and then $5 \times 10^5$ cells were dispensed into tubes. As a result, the peripheral blood lymphocytes having occupancy rates of nivolumab of 0%, 25%, 50%, 75%, and 100% were obtained.

Subsequently, Human BD Fc Block (0.5 $\mu$g/mL, BD Biosciences) was added to the individual cells and reacted at 4°C for 10 minutes. Subsequently, 1 μg/mL of an allophycocyanin (APC)-labeled anti-human CD3 antibody, 4 μg/mL of the Alexa Fluor 488-labeled anti-PD-1 antibody (clone EH12.2H7, Biolegend Inc.), and 10 μg/mL of the PE-labeled anti-PD-1 antibody (clone MIH4, eBioscience Inc.) were and reacted at 4°C for 20 minutes.

Subsequently, the individual cells were washed twice with 600 μL of PBS. Subsequently, the binding of the Alexa Fluor 488-labeled anti-PD-1 antibody (clone EH12.2H7) and the PE-labeled anti-PD-1 antibody (clone MIH4) to the individual cells were analyzed using the flow cytometer (model "FACSVerse", BD Bioscience).

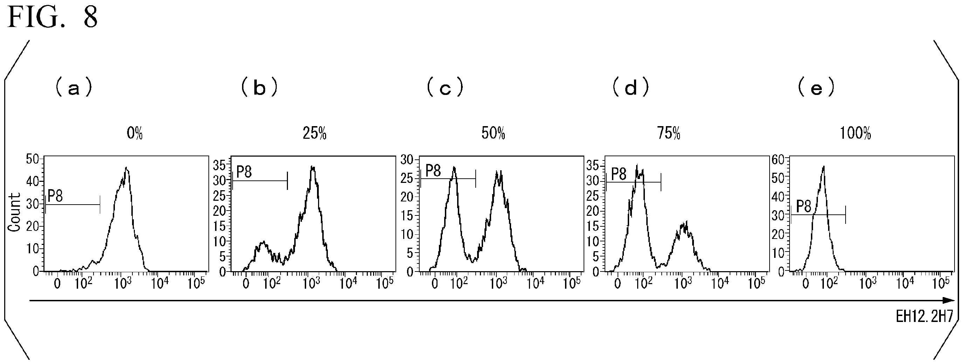

FIGS. 8(A) to 8(E) are graphs showing the analysis results. In FIGS. 8(A) to 8(E), the results obtained by analyzing only T cells by gating CD3-positive cells are shown. FIGS. 8(A) to 8(E) show the results obtained in the peripheral blood lymphocytes prepared so that the occupancy rates of nivolumab were respectively 0%, 25%, 50%, 75%, and 100%. In FIGS. 8(A) to 8(E), the vertical axis represents the number of cells, and the horizontal axis represents the fluorescence intensity of Alexa Fluor 488.

From the results, it was recognized that the fluorescence intensity of Alexa Fluor 488 tended to decrease as the occupancy rate of nivolumab increased.

In addition, based on the results obtained by analyzing with the flow cytometer, the occupancy rate of nivolumab was calculated by following Expression (3A).

$$\text{Occupancy rate of nivolumab (\%)} = (1 - (\text{Proportion of cells to which anti-PD-1}$$

$$\text{antibody (EH12.2H7) has bound/Proportion of cells to which anti-PD-1 antibody (clone}$$

$$\text{MIH4)) has bound}) \times 100 \cdots (3A)$$

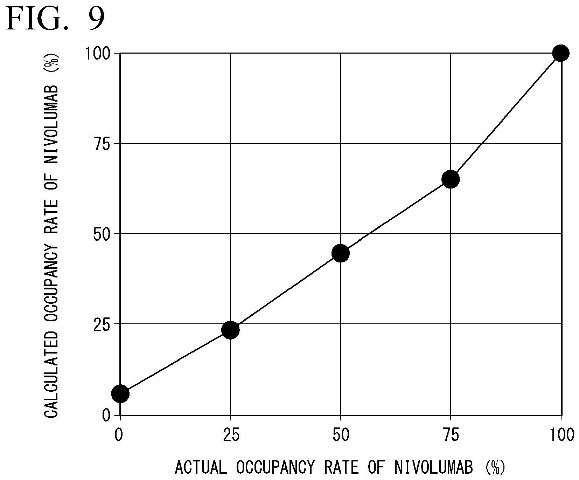

FIG. 9 is a graph showing an occupancy rate of nivolumab calculated by above Expression (3A) and an actual occupancy rate of nivolumab. From the result, it was confirmed that the occupancy rate of nivolumab calculated by above Expression (3A) well matched the actual occupancy rate of nivolumab. This result indicates that the occupancy rate of nivolumab in human T cells can be measured by the method of this Experimental Example.

[Experimental Example 6]

(Measurement 3 of occupancy rate of nivolumab)

The anti-PD-1 antibody (clone EH12.2H7) and the anti-PD-1 antibody (clone MIH4) were used to measure an occupancy rate of nivolumab in T cells in lymphocytes derived from the body cavity fluid of a patient administered with nivolumab. The pleural effusion fluid and the alveolar lavage fluid were used as the body cavity fluid.

First, lymphocytes derived from the pleural effusion fluid of a patient at 12 days after administration of nivolumab, and lymphocytes derived from the alveolar lavage fluid of the patient at 21 days after administration of nivolumab were collected. For comparison, lymphocytes derived from pleural effusion fluid of a patient who was not administered with nivolumab were also collected. Subsequently, $5 \times 10^5$ cells of the lymphocyte each were dispensed into tubes.

Subsequently, Human BD Fc Block (0.5 μg/mL, BD Biosciences) was added to the individual cells and reacted at 4°C for 10 minutes. Subsequently, 1 μg/mL of the APC-labeled anti-human CD3 antibody, 4 μg/mL of the Alexa Fluor 488-labeled anti-PD-1 antibody (clone EH12.2H7, Biolegend Inc.), and 10 μg/mL of the PE-labeled anti-PD-1 antibody (clone MIH4, eBioscience Inc.) were added and reacted at 4°C for 20 minutes.

Subsequently, the individual cells were washed twice with 600 μL of PBS. Subsequently, the binding of the Alexa Fluor 488-labeled anti-PD-1 antibody (clone EH12.2H7) and the PE-labeled anti-PD-1 antibody (clone MIH4) to the individual cells were analyzed using the flow cytometer (model "FACSVerse", BD Bioscience).

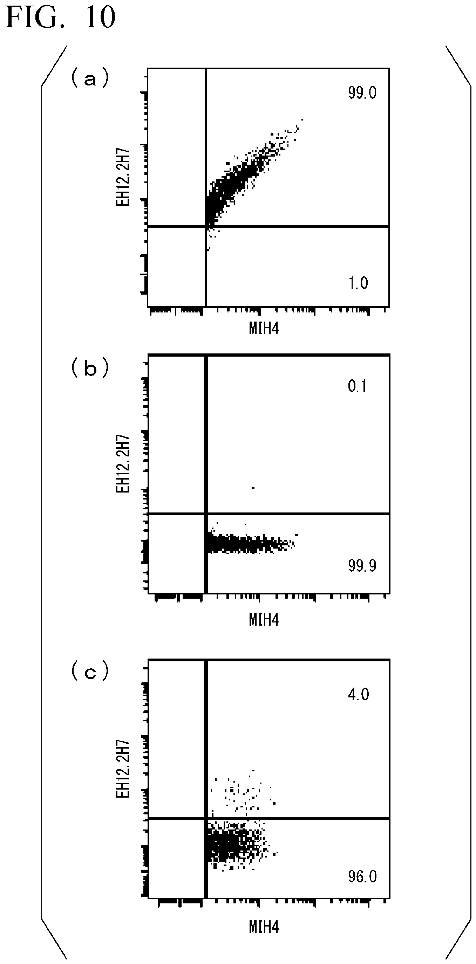

FIGS. 10(A) to 10(C) are graphs showing the analysis results. In FIGS. 10(A) to 10(E), the results obtained by analyzing only T cells by gating CD3-positive cells are shown. FIG. 10(A) is the result obtained by analyzing T cells derived from the pleural effusion fluid of a patient not administered with nivolumab, FIG. 10(B) is the result obtained by analyzing T cells derived from the pleural effusion fluid of the patient at 12 days after administration of nivolumab, and FIG. 10(C) is the result obtained by analyzing T cells derived from the alveolar lavage fluid of the patient at 21 days after the administration of nivolumab. In FIGS. 10(A) to 10(C), the vertical axis represents the fluorescence intensity of Alexa Fluor 488, and the horizontal axis represents the fluorescence intensity of PE.

In addition, based on the results obtained by analyzing with the flow cytometer, the occupancy rate of nivolumab was calculated by following Expression (3A).

$$\text{Occupancy rate of nivolumab (\%)} = (1 - (\text{Proportion of cells to which anti-PD-1}$$

$$\text{antibody (EH12.2H7) has bound/Proportion of cells to which anti-PD-1 antibody (clone}$$

$$\text{MIH4)) has bound)} \times 100 \cdots \text{(3A)}$$

From the results, as shown in FIG. 10(A), the occupancy rate of nivolumab was calculated to be 1% in T cells derived from the pleural effusion fluid of the patient not administered with nivolumab. This value was considered to be an error. In contrast, as shown in FIG. 10(B), the occupancy rate of nivolumab was calculated to be 99.9% in T cells derived from the pleural effusion fluid of the patient at 12 days after administration of nivolumab. In addition, as shown in FIG. 10(C), the occupancy rate of nivolumab was calculated to be 96.0% in T cells derived from the alveolar lavage fluid of the patient at 21 days after administration of nivolumab.

This result indicates that the occupancy rate of nivolumab in T cells of a patient during actual treatment can be measured by the method of this Experimental Example.

[Experimental Example 7]

(Measurement of occupancy rate of pembrolizumab)

The anti-PD-1 antibody (clone EH12.2H7) and the anti-PD-1 antibody (clone MIH4) were used to measure an occupancy rate of pembrolizumab in the MIT9 cell line.

First, 10 μg/mL of pembrolizumab was added to the MIT9 cell line and reacted at 4°C for 30 minutes. Subsequently, cells were washed three times with 600 μL of PBS. Subsequently, a MIT9 cell line not reacted with pembrolizumab was mixed with the MIT9 cell line reacted with pembrolizumab so that the proportion of the MIT9 cell line reacted with pembrolizumab was 0%, 25%, 50%, 75%, and 100%, and then $2 \times 10^5$ cells of the mixed cell lines were dispensed into tubes. As a result, MIT9 cell lines each having occupancy rates of pembrolizumab of 0%, 25%, 50%, 75%, and 100% were obtained.

Subsequently, 4 μg/mL of the Alexa Fluor 488-labeled anti-PD-1 antibody (clone EH12.2H7, Biolegend Inc.) and 10 μg/mL of the PE-labeled anti-PD-1 antibody (clone MIH4, eBioscience Inc.) were added to the individual cells and reacted at 4°C for 20 minutes.

Subsequently, the individual cells were washed twice with 600 μL of PBS. Subsequently, the binding of the Alexa Fluor 488-labeled anti-PD-1 antibody (clone EH12.2H7) and the PE-labeled anti-PD-1 antibody (clone MIH4) to the individual cells were analyzed using the flow cytometer (model "FACSVerse", BD Bioscience).

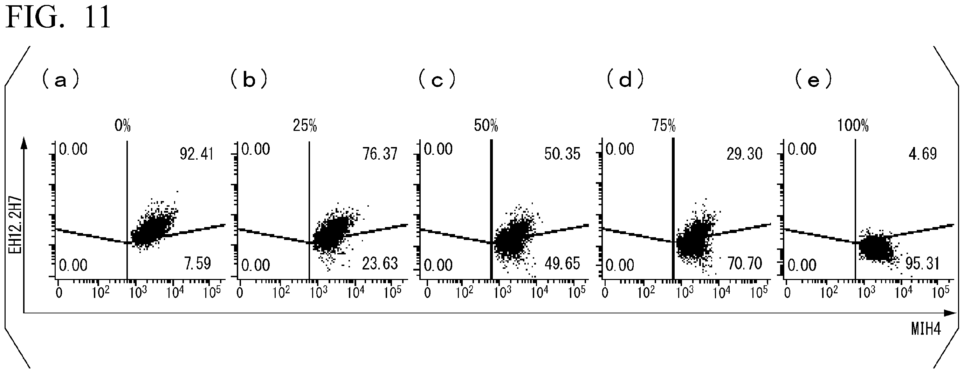

FIGS. 11(A) to 11(E) are graphs showing the analysis results. FIGS. 11(A) to 11(E) show the results obtained in the MIT9 cell lines prepared so that the occupancy rates of pembrolizumab were respectively 0%, 25%, 50%, 75%, and 100%. In FIGS. 11(A) to 11(E), the vertical axis represents the fluorescence intensity of Alexa Fluor 488, and the horizontal axis represents the fluorescence intensity of PE.

From the results, it was recognized that the fluorescence intensity of Alexa Fluor 488 tended to decrease as the occupancy rate of pembrolizumab increased. On the other hand, it was recognized that the fluorescence intensity of PE tended not to change even in a case where the occupancy rate of pembrolizumab changed.

Subsequently, based on the results of FIGS. 11(A) to 11(E), the occupancy rate of pembrolizumab was calculated by following Expression (3B).

$$\text{Occupancy rate of pembrolizumab (\%)} = (1 - (\text{Proportion of cells to which}$$

$$\text{anti-PD-1 antibody (EH12.2H7) has bound/Proportion of cells to which anti-PD-1}$$

$$\text{antibody (clone MIH4)) has bound)} \times 100 \cdots \text{(3B)}$$

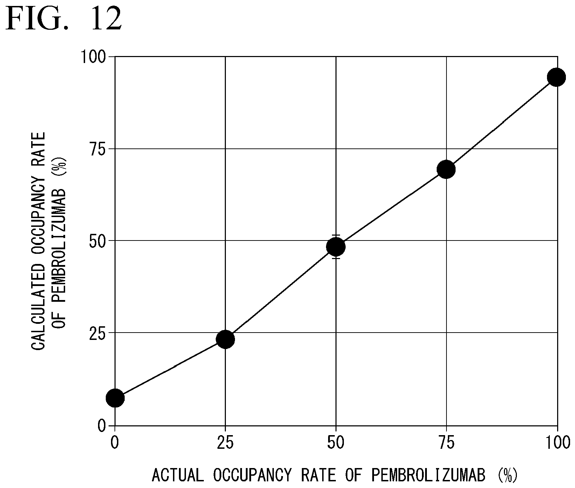

FIG. 12 is a graph showing an occupancy rate of pembrolizumab calculated by above Expression (3B) and an actual occupancy rate of pembrolizumab. From the result, it was confirmed that the occupancy rate of pembrolizumab calculated by above Expression (3B) well matched the actual occupancy rate of pembrolizumab. This result indicates that the occupancy rate of pembrolizumab can be measured by the method of this Experimental Example.

[Experimental Example 8]

(Measurement 4 of occupancy rate of nivolumab)

The anti-PD-1 antibody (clone EH12.2H7) and the anti-PD-1 antibody (clone MIH4) were used to measure an occupancy rate of nivolumab in T cells in peripheral blood lymphocytes using blood samples of two lung cancer patients (hereinafter referred to as "patient 1" and "patient 2") not administered with nivolumab as a sample.

First, peripheral blood lymphocytes were collected from the blood samples of each patient. Subsequently, $5 \times 10^5$ cells of the lymphocyte each were dispensed into tubes.

Subsequently, Human BD Fc Block (0.5 $\mu$g/mL, BD Biosciences) was added to the individual cells and reacted at 4°C for 10 minutes. Subsequently, 1 $\mu$g/mL of the APC-labeled anti-human CD3 antibody, 4 $\mu$g/mL of the Alexa Fluor 488-labeled anti-PD-1 antibody (clone EH12.2H7, Biolegend Inc.), and 10 $\mu$g/mL of the PE-labeled anti-PD-1 antibody (clone MIH4, eBioscience Inc.) were added and reacted at 4°C for 20 minutes.

Subsequently, the individual cells were washed twice with 600 $\mu$L of PBS. Subsequently, the binding of the Alexa Fluor 488-labeled anti-PD-1 antibody (clone EH12.2H7) and the PE-labeled anti-PD-1 antibody (clone MIH4) to the individual cells were analyzed using the flow cytometer (model "FACSVerse", BD Bioscience).

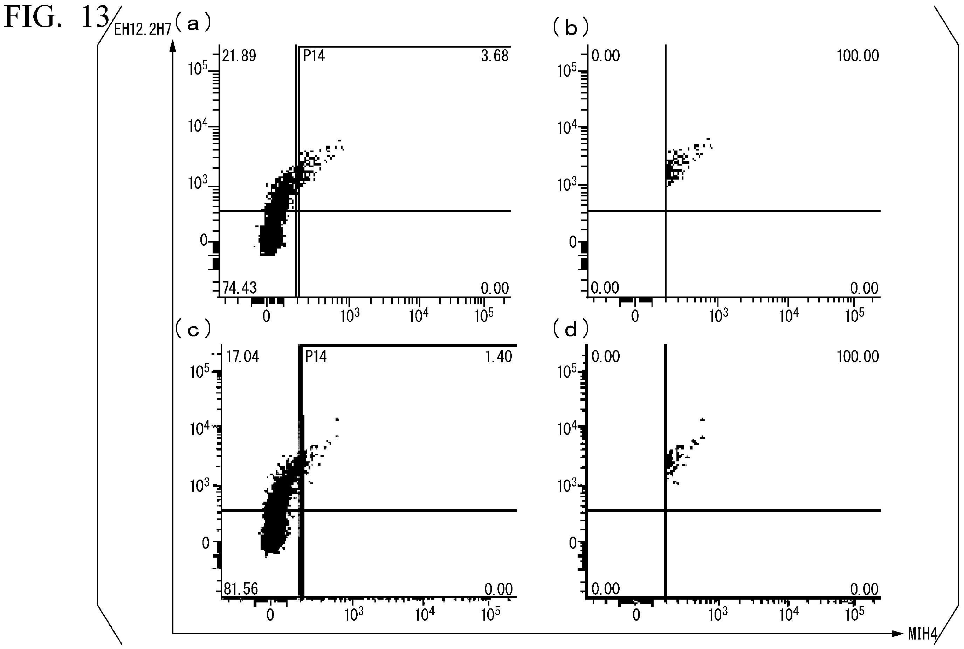

FIGS. 13(A) to 13(D) are graphs showing the analysis results. FIG. 13(A) shows the result obtained by analyzing peripheral blood lymphocytes of patient 1, and FIG. 13(B) shows the result obtained by analyzing peripheral blood lymphocytes of patient 2. FIG. 13(C) is a graph showing the result obtained by analyzing only T cells of patient 1 by gating CD3-positive cells. FIG. 13(D) is a graph showing the result obtained by analyzing only T cells of patient 2 by gating CD3-positive cells. In FIGS. 13(A) to 13(D), the vertical axis represents the fluorescence intensity of Alexa Fluor 488, and the horizontal axis represents the fluorescence intensity of PE.

Subsequently, based on the results of FIGS. 13(A) and 13(D), the occupancy rate of nivolumab was calculated by above Expression (3A). From the results, the occupancy rates of nivolumab in T cells were calculated to be 0% in all patients.

This result indicates that the occupancy rate of nivolumab in T cells of a patient using a blood sample as a sample can be measured by the method of this Experimental Example.

[Experimental Example 9]

(Measurement 5 of occupancy rate of nivolumab)

The anti-PD-1 antibody (clone EH12.2H7) and the anti-PD-1 antibody (clone MIH4) were used to measure an occupancy rate of nivolumab in T cells in peripheral blood lymphocytes using a blood sample of a renal carcinoma patient administered with nivolumab as a sample. This patient received nine courses of treatment with nivolumab but developed drug-induced pneumonia, and thus treatment with nivolumab was discontinued. Two weeks with one administration of nivolumab correspond to one course.

First, at three weeks after the ninth administration of nivolumab, a blood sample was collected from the patient. Subsequently, peripheral blood lymphocytes were collected from the blood sample. Subsequently, $5 \times 10^5$ cells of the lymphocyte each were dispensed into tubes.

Subsequently, Human BD Fc Block (0.5 $\mu$g/mL, BD Biosciences) was added to the individual cells and reacted at 4°C for 10 minutes. Subsequently, 1 $\mu$g/mL of the APC-labeled anti-human CD3 antibody, 4 $\mu$g/mL of the Alexa Fluor 488-labeled anti-PD-1 antibody (clone EH12.2H7, Biolegend Inc.), and 10 $\mu$g/mL of the PE-labeled anti-PD-1 antibody (clone MIH4, eBioscience Inc.) were added and reacted at 4°C for 20 minutes.

Subsequently, the cells were washed twice with 600 $\mu$L of PBS. Subsequently, the binding of the Alexa Fluor 488-labeled anti-PD-1 antibody (clone EH12.2H7) and the PE-labeled anti-PD-1 antibody (clone MIH4) to the cells were analyzed using the flow cytometer (model "FACSVerse", BD Bioscience).

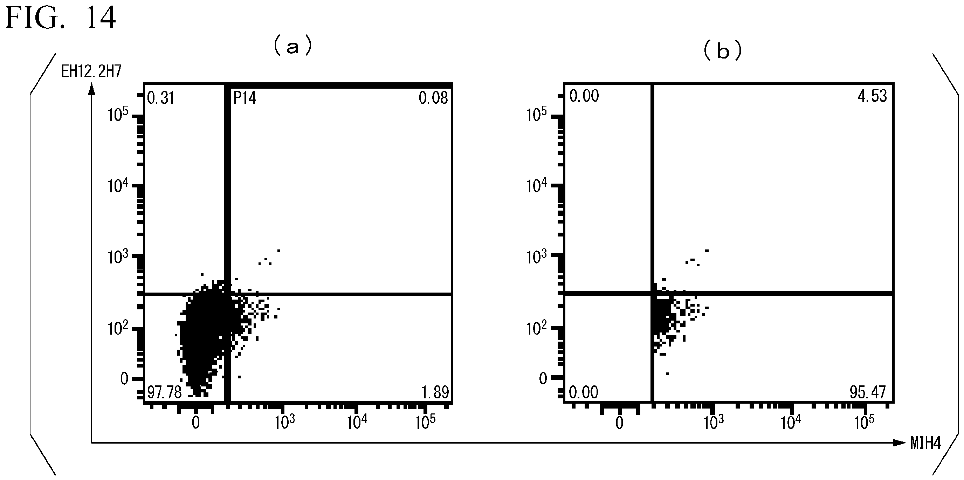

FIGS. 14(A) and 14(B) are graphs showing the analysis results. FIG. 14(A) shows the result obtained by analyzing peripheral blood lymphocytes. FIG. 14(B) is a graph showing the result obtained by analyzing only T cells of the patient by gating CD3-positive cells. In FIGS. 13(A) and 13(B), the vertical axis represents the fluorescence intensity of Alexa Fluor 488, and the horizontal axis represents the fluorescence intensity of PE.

Subsequently, based on the result of FIG. 14(B), the occupancy rate of nivolumab was calculated by above Expression (3A). From the result, the occupancy rate of nivolumab in T cells was calculated to be 95.47%.

This result further supports that the occupancy rate of nivolumab in T cells of a patient using a blood sample as a sample can be measured by the method of this Experimental Example.

[Experimental Example 10]

(Measurement 2 of occupancy rate of pembrolizumab)

The anti-PD-1 antibody (clone EH12.2H7) and the anti-PD-1 antibody (clone MIH4) were used to measure an occupancy rate of pembrolizumab in T cells in peripheral blood lymphocytes using a blood sample of a lung cancer patient administered with pembrolizumab as a sample. This patient received two courses of treatment with pembrolizumab but developed drug-induced pneumonia, and thus treatment with pembrolizumab was discontinued. Three weeks with one administration of pembrolizumab correspond to one course.

First, at three weeks after the second administration of pembrolizumab, a blood sample was collected from the patient. Subsequently, peripheral blood lymphocytes were collected from the blood sample. Subsequently, $5 \times 10^5$ cells of the lymphocyte each were dispensed into tubes.

Subsequently, Human BD Fc Block (0.5 $\mu$g/mL, BD Biosciences) was added to the individual cells and reacted at 4°C for 10 minutes. Subsequently, 1 $\mu$g/mL of the APC-labeled anti-human CD3 antibody, 4 $\mu$g/mL of the Alexa Fluor 488-labeled anti-PD-1 antibody (clone EH12.2H7, Biolegend Inc.), and 10 $\mu$g/mL of the PE-labeled anti-PD-1 antibody (clone MIH4, eBioscience Inc.) were added and reacted at 4°C for 20 minutes.

Subsequently, the cells were washed twice with 600 $\mu$L of PBS. Subsequently, the binding of the Alexa Fluor 488-labeled anti-PD-1 antibody (clone EH12.2H7) and the PE-labeled anti-PD-1 antibody (clone MIH4) to the cells were analyzed using the flow cytometer (model "FACSVerse", BD Bioscience).

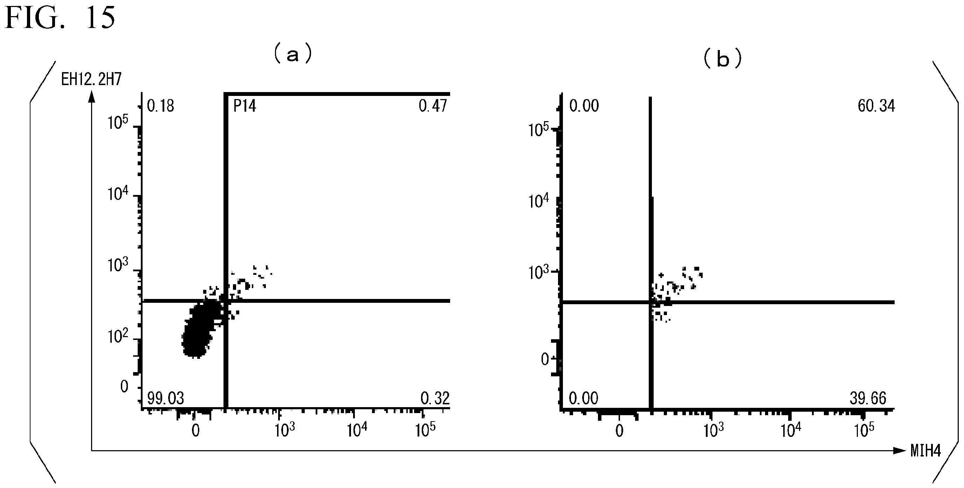

FIGS. 15(A) and 15(B) are graphs showing the analysis results. FIG. 15(A) shows the result obtained by analyzing peripheral blood lymphocytes. FIG. 15(B) is a graph showing the result obtained by analyzing only T cells of the patient by gating CD3-positive cells. In FIGS. 15(A) and 15(B), the vertical axis represents the fluorescence intensity of Alexa Fluor 488, and the horizontal axis represents the fluorescence intensity of PE.

Subsequently, based on the result of FIG. 15(B), the occupancy rate of pembrolizumab was calculated by above Expression (3B). From the result, the occupancy rate of pembrolizumab in T cells was calculated to be 39.66%.

This result indicates that the occupancy rate of pembrolizumab in T cells of a patient using a blood sample as a sample can be measured by the method of this Experimental Example.

Industrial Applicability

According to the present invention, a technique for simply measuring the dynamics of a specific binding substance can be provided.

**Claims**

1. A method for measuring an occupancy rate of a first specific binding substance in a cell population, the method comprising:

bringing a second specific binding substance into contact with the cell population;
bringing a third specific binding substance into contact with the cell population;
counting the number of cells to which the second specific binding substance binds; and
counting the number of cells to which the third specific binding substance binds,
wherein all of the first, the second, and the third specific binding substances are specific binding substances that bind to a target cell surface protein,
the occupancy rate is a proportion of cells to which the first specific binding substance binds, in cells expressing the target cell surface protein,
the second specific binding substance is a specific binding substance that competes with the first specific binding substance,
the third specific binding substance is a specific binding substance that binds to the target cell surface protein without competing with the first specific binding substance, and
a value calculated by Expression (1) is the occupancy rate:

$$\text{Occupancy rate (\%) of the first specific binding substance} = (1 - (\text{Number of}$$

$$\text{cells to which the second specific binding substance binds/Number of cells to which the}$$

$$\text{third specific binding substance binds})) \times 100 \cdots (1).$$

2. The method according to claim 1, wherein the bringing of the second specific binding substance into contact and the bringing of the third specific binding substance into contact are performed simultaneously.

3. The method according to claim 1 or 2, wherein the counting of the number of cells to which the second specific binding substance binds and the counting of the number of cells to which the third specific binding substance binds are performed simultaneously.

4. The method according to any one of claims 1 to 3, wherein the first specific binding substance is an antibody.

5. The method according to claim 4, wherein the target cell surface protein is human programmed death-1 (PD-1), and the first specific binding substance is an anti-human PD-1 antibody.

6. The method according to claim 5, wherein the first specific binding substance is nivolumab or pembrolizumab, the second specific binding substance is an anti-human PD-1 monoclonal antibody (clone EH12.2H7), and the third specific binding substance is an anti-human PD-1 monoclonal antibody (clone MIH4).

7. A kit for measuring an occupancy rate of a first specific binding substance in a cell population, the kit comprising:

   a second specific binding substance; and
   a third specific binding substance,
   wherein all of the first, the second, and the third specific binding substances are specific binding substances that bind to a target cell surface protein,
   the occupancy rate is a proportion of cells to which the first specific binding substance binds, in cells expressing the target cell surface protein,
   the second specific binding substance is a specific binding substance that competes with the first specific binding substance, and
   the third specific binding substance is a specific binding substance that binds to the target cell surface protein without competing with the first specific binding substance.

8. The kit according to claim 7, wherein the first specific binding substance is an antibody.

9. The kit according to claim 8, wherein the target cell surface protein is human PD-1, and the first specific binding substance is an anti-human PD-1 antibody.

10. The kit according to claim 9, wherein the first specific binding substance is nivolumab or pembrolizumab, the second specific binding substance is an anti-human PD-1 monoclonal antibody (clone EH12.2H7), and the third specific binding substance is an anti-human PD-1 monoclonal antibody (clone MIH4).

11. A method for obtaining data for optimizing an administration interval or a dosage of an antibody drug to a patient, the method comprising:

   bringing a second specific binding substance into contact with cells derived from the patient administered with the antibody drug;
   bringing a third specific binding substance into contact with the cells derived from the patient administered with the antibody drug;
   counting the number of cells to which the second specific binding substance binds; and
   counting the number of cells to which the third specific binding substance binds,
   wherein both the second and the third specific binding substances are specific binding substances that bind to a target protein of the antibody drug,
   the second specific binding substance is a specific binding substance that competes with the antibody drug,

the third specific binding substance is a specific binding substance that binds to the target protein of the antibody drug without competing with the antibody drug, and

an occupancy rate of the antibody drug calculated by Expression (2) is the data for optimizing the administration interval or the dosage of the antibody drug to the patient:

$$\text{Occupancy rate (\%) of the antibody drug} = (1 - (\text{Number of cells to which the second specific binding substance binds/Number of cells to which the third specific binding substance binds})) \times 100 \cdots (2).$$

12. The method according to claim 11, wherein the bringing of the second specific binding substance into contact and the bringing of the third specific binding substance into contact are performed simultaneously.

13. The method according to claim 11 or 12, wherein the counting of the number of cells to which the second specific binding substance binds and the counting of the number of cells to which the third specific binding substance binds are performed simultaneously.

14. The method according to any one of claims 11 to 13, wherein the occupancy rate being higher than a reference value indicates the administration interval of the antibody drug to the patient to be lengthened or the dosage to be reduced, and the occupancy rate being lower than the reference value indicates the administration interval of the antibody drug to the patient to be shortened or the dosage to be increased.

15. A method for obtaining data for optimizing an administration interval or a dosage of nivolumab or pembrolizumab to a patient, the method comprising:

bringing a second specific binding substance into contact with cells derived from a patient administered with nivolumab or pembrolizumab;
bringing a third specific binding substance into contact with the cells derived from the patient administered with nivolumab or pembrolizumab;
counting the number of cells to which the second specific binding substance binds; and
counting the number of cells to which the third specific binding substance binds,
wherein both the second and the third specific binding substances are specific binding substances that bind to human PD-1,
the second specific binding substance is a specific binding substance that competes with nivolumab or pembrolizumab,
the third specific binding substance is a specific binding substance that binds to human PD-1 without competing with nivolumab or pembrolizumab, and
an occupancy rate of nivolumab or pembrolizumab calculated by Expression (3) is the data for optimizing the administration interval or the dosage of nivolumab or pembrolizumab to the patient:

$$\text{Occupancy rate (\%) of nivolumab or pembrolizumab} = (1 - (\text{Number of cells to which the second specific binding substance binds/Number of cells to which the third specific binding substance binds})) \times 100 \cdots (3).$$

16. The method according to claim 15, wherein the bringing of the second specific binding substance into contact and the bringing of the third specific binding substance into contact are performed simultaneously.

17. The method according to claim 15 or 16, wherein the counting of the number of cells to which the second specific binding substance binds and the counting of the number of cells to which the third specific binding substance binds are performed simultaneously.

18. The method according to any one of claims 15 to 17, wherein the occupancy rate being higher than a reference value indicates the administration interval of nivolumab or pembrolizumab to the patient to be lengthened or the dosage to be reduced, and the occupancy rate being lower than the reference value indicates the administration interval of nivolumab or pembrolizumab to the patient to be shortened or the dosage to be increased.

19. The method according to any one of claims 15 to 18, wherein the second specific binding substance is an anti-human PD-1 monoclonal antibody (clone EH12.2H7), and the third specific binding substance is an anti-human PD-1 monoclonal antibody (clone MIH4).

FIG. 1

FIG. 2

# FIG. 3

# FIG. 4

FIG. 5

EP 3 667 318 A1

FIG. 6

FIG. 7

EP 3 667 318 A1

FIG. 8

(a) 0%
(b) 25%
(c) 50%
(d) 75%
(e) 100%

FIG. 9

# FIG. 10

FIG. 11

EP 3 667 318 A1

FIG. 12

FIG. 13

# FIG. 14

（ a ）

EH12.2H7

| 0.31 | P14 | 0.08 |

| 97.78 | | 1.89 |

MIH4

（ b ）

| 0.00 | | 4.53 |

| 0.00 | | 95.47 |

# FIG. 15

（a）

（b）

EP 3 667 318 A1

## INTERNATIONAL SEARCH REPORT

| | International application No. |
|---|---|
| | PCT/JP2018/029390 |

### A. CLASSIFICATION OF SUBJECT MATTER
Int.Cl. G01N33/53(2006.01)i, A61K39/395(2006.01)i, A61P35/00(2006.01)i

According to International Patent Classification (IPC) or to both national classification and IPC

### B. FIELDS SEARCHED

Minimum documentation searched (classification system followed by classification symbols)
Int.Cl. G01N33/53, A61K39/395, A61P35/00

Documentation searched other than minimum documentation to the extent that such documents are included in the fields searched
| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| X<br>Y<br>A | JP 2016-536314 A (SANOFI) 24 November 2016, paragraphs [0153]-[0192] & US 2015/0118251 A1, paragraphs [0156]-[0195] & WO 2015/066450 A1 | 1-4, 7-8, 11-14<br>5, 9, 15-18<br>6, 10, 19 |
| X<br>Y | WO 2004/054616 A1 (ONO PHARMACEUTICAL CO., LTD.) 01 July 2004, claims 9-12, examples 2, 5 & US 2006/0251651 A1, claims 9-12, examples 2, 5 & EP 1570860 A1 | 1-3, 7<br>4-5, 8-9, 11-18 |
| Y | BRAHMER, J. R. et al., "Phase I Study of Single-Agent Anti-Programmed Death-1(MDX-1106) in Refractory Solid Tumors: Safety, Clinical Activity, Pharmacodynamics, and Immunologic Correlates", JOURNAL OF CLINICAL ONCOLOGY, 2010, vol. 28, no. 19, pp. 3167-3175 | 4-5, 8-9, 11-18 |

☒ Further documents are listed in the continuation of Box C. ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 24 October 2018 (24.10.2018) | 06 November 2018 (06.11.2018) |

| Name and mailing address of the ISA/<br>Japan Patent Office<br>3-4-3, Kasumigaseki, Chiyoda-ku,<br>Tokyo 100-8915, Japan | Authorized officer<br><br><br>Telephone No. |
|---|---|

Form PCT/ISA/210 (second sheet) (January 2015)

37

## INTERNATIONAL SEARCH REPORT

International application No.

PCT/JP2018/029390

C (Continuation). DOCUMENTS CONSIDERED TO BE RELEVANT

| Category* | Citation of document, with indication, where appropriate, of the relevant passages | Relevant to claim No. |
|---|---|---|
| A | JP 2015-534949 A (THE BRIGHAM AND WOMEN'S HOSPITAL, INC.) 07 December 2015, paragraph [0056] & US 2015/0273056 A1, paragraph [0071] & WO 2014/059251 A1 | 1-19 |
| A | US 2016/0251436 A1 (AMIRINA, N. et al.) 01 September 2016, table 3 & WO 2016/106159 A1 | 1-19 |
| A | SIGURSKJOLD, B. W., "Exact Analysis of Competition Ligand Binding by Displacement Isothermal Titration Calorimetry", Analytical Biochemistry, 2000, vol. 277, pp. 260-266 | 1-19f |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017152735 A

**Non-patent literature cited in the description**

- **BRAHMER J. R.** Phase I study of single-agent anti-programmed death-1 (MDX-1106) in refractory solid tumors: safety, clinical activity, pharmacodynamics, and immunologic correlates. *J. Clin. Oncol.,* 2010, vol. 28 (19), 3167-3175